# EUROPÄISCHE PATENTSCHRIFT

(11) **EP 2 720 616 B1**
(45) Veröffentlichungstag und Bekanntmachung des Hinweises auf die Patenterteilung: **24.02.2021**
(21) Anmeldenummer: 11758105.8
(22) Anmeldetag: 14.06.2011
(51) Int. Cl.: A61B 10/00

(54) **VORRICHTUNG ZUR ENTNAHME UND AUFBEREITUNG EINER PROBE**
DEVICE FOR TAKING AND PROCESSING A SAMPLE
DISPOSITIF POUR LE PRÉLÈVEMENT ET LE TRAITEMENT D'UN ÉCHANTILLON

(43) Veröffentlichungstag der Anmeldung: 23.04.2014
(73) Patentinhaber: Frost Diagnostika GmbH, 67166 Otterstadt (DE)
(72) Erfinder: DITTMANN, Ludwig, 14163 Berlin (DE); FROST, Gabriele, Maria, 67346 Speyer (DE)
(74) Vertreter: Fleuchaus, Andrea
(86) Internationale Anmeldenummer: PCT/DE2011/075132
(87) Internationale Veröffentlichungsnummer: WO 2012/171511

(56) Entgegenhaltungen:
- EP-A1- 1 986 006
- EP-A1- 2 287 331
- DE-B1- 2 835 358
- DE-U1-202009 015 624
- US-A1- 2004 179 976

## Beschreibung

Die Erfindung betrifft eine Vorrichtung zur Entnahme und Aufbereitung einer Probe zur qualitativen und quantitativen Bestimmung von in dieser enthaltenen Substanzen, insbesondere einer Stuhlprobe für die Stuhluntersuchung, die einen Probenahmestab mit einer als Probenahmeraum ausgebildeten Entnahmestruktur zur Aufnahme einer bestimmten Probemenge und ein den Probenahmestab aufnehmendes Aufnahmeröhrchen, das einen mit einer Flüssigkeit gefüllten Mischraum zur Konservierung, Auflösung und Aufbereitung der in dem Probenahmeraum befindlichen Probe aufweist, umfasst.

Der menschliche Stuhl kann eine Vielzahl von Substanzen und Erregern enthalten, deren Art und prozentualer Anteil im Stuhl für die Erkennung und Behandlung vieler Krankheiten von ausschlaggebender Bedeutung ist. Beispielsweise können vom Körper aufgenommene giftige oder radioaktive Stoffe, Erreger von Entzündungen, Parasiten und andere Substanzen wie beispielsweise das für die Abwehr körperfremder Stoffe und bei allergischen Reaktionen eine wichtige Rolle spielende Histamin durch Stuhluntersuchungen erfasst werden.

Nach dem Erfassen der im Stuhl enthaltenen Substanzen in einem ersten Untersuchungsschritt ist eine verlässliche diagnostische Abklärung der auf die festgestellte(n) Substanz (en) zurückzuführenden Beschwerden nur durch eine in einem zweiten Untersuchungsschritt durchgeführte genaue Bestimmung ihres prozentualen Anteils im Stuhl möglich. Beide Untersuchungsschritte können mit einer in der DE 20 2009 015 624.9 beschriebenen Stuhlprobenahmevorrichtung der eingangs erwähnten Art mit einer einzigen Probenahme durchgeführt werden, da bei der Probenahme mit dem am Stuhlentnahmestab ausgebildeten Probenahmeraum ein ganz bestimmtes Stuhlvolumen entnommen wird und in der im Aufnahmeröhrchen befindlichen Flüssigkeit gelöst wird. Aufgrund der in der Flüssigkeit gelösten definierten Stuhlmenge können mit ein und derselben Probe in einem ersten Test zum einen die darin enthaltenen Substanzen als solche festgestellt werden und zum anderen kann in einem gegebenenfalls erforderlichen zweiten Test aber auch deren prozentualer Anteil im Stuhl ermittelt werden.

Die aus der DE 20 2009 015 624.9 bekannte Vorrichtung zur Entnahme von Stuhlproben ist insofern nachteilig, als es bei einem harten bis sehr harten Stuhl (dem sogenannten Betonstuhl) Schwierigkeiten bereitet, diesen Stuhl mit der im Aufnahmeröhrchen befindlichen Flüssigkeit vollständig aus dem Probenahmeraum herauszulösen und in der Flüssigkeit vollständig aufzulösen und gegebenenfalls erst während der Stuhlauflösung für die Untersuchung erforderliche Reagenzien in die Flüssigkeit einzubringen.

Bei flüssigen Stühlen ist auch die Entnahme eines vorgegebenen Stuhlvolumens schwierig. Aus US 2004/179976 A1 DE 28 35 358 B1, EP 2287 331 A1 und EP 1 986 006 sind weitere Vorrichtungen zur Entnahme von Stuhlproben bekannt.

Der Erfindung liegt die Aufgabe zugrunde, eine Vorrichtung der eingangs erwähnten Art anzugeben, mit der eine flüssige bis sehr feste Probe für eine präzise qualitative und quantitative Feststellung von in dieser enthaltenen Substanzen entnommen und aufbereitet werden kann. Erfindungsgemäß wird die Aufgabe mit einer gemäß den Merkmalen des Patentanspruchs 1 ausgebildeten Vorrichtung gelöst.

Weitere vorteilhafte und zweckmäßige Merkmale der Erfindung sind Gegenstand der Unteransprüche.

Ausgehend von der in der Beschreibungseinleitung angegebenen Vorrichtung zur Entnahme und Aufbereitung einer Stuhlprobe zur qualitativen und quantitativen Bestimmung von in dieser enthaltenen Substanzen besteht der Kern der Erfindung in der Ausbildung des Probenahmestabes als beidseitig offener, rohrartiger Hohlkörper mit einem in diesem verschiebbar und abdichtend geführten Ausstoßstempel zum Ausstoßen der Probe aus dem am freien Ende des Probenahmestabes als Kapillare ausgebildeten Probenahmeraum in den mit einer Flüssigkeit gefüllten Mischraum. Insbesondere eine sehr feste, jetzt im Mischraum befindliche Probe kann schnell und vollständig in der Flüssigkeit gelöst werden, so dass bei der anschließenden qualitativen und quantitativen Untersuchung ein- und desselben Probematerials zuverlässige Ergebnisse erzielt werden können. Die Ausbildung des Probenahmeraums als enge Kapillare ermöglicht aufgrund der Kapillarkräfte auch die zuverlässige Entnahme von Proben in einer bestimmten Menge aus einem weichen bis flüssigen Probematerial, zum Beispiel Flüssigstuhl. Die Vorrichtung kann gleichermaßen zur Entnahme und Aufbereitung von Lebensmittelproben in einem bestimmten Volumen genutzt werden.

Das stirnseitige distale Ende des Ausstoßstempels bildet den Boden des Probenahmeraums.

Gemäß einem noch anderen Merkmal der Erfindung kann der distale Endabschnitt des Ausstoßstempels von mindestens einem - nach dem Ausstoßen der Probe in den Mischraum als Mischhilfe fungierenden - Kügelchen gebildet sein. Eine noch an einem Kügelchen haftende Probe löst sich leichter von dem im Mischraum durch Schütteln des Aufnahmeröhrchens beweglichen Kügelchen. Außerdem bewirkt das in der Flüssigkeit bewegte Kügelchen eine schnellere und bessere Durchmischung.

In Ausgestaltung der Erfindung kann das Kügelchen mit einer in der Flüssigkeit löslichen Reaktionssubstanz beschichtet sein, die sich nach dem Ausstoßen der Probe in den Mischraum in der Flüssigkeit löst und die in der Flüssigkeit gelöste Probe für eine anschließende Untersuchung aufbereitet.

Andererseits kann auch ein zwischen dem Kügelchen und dem Ausstoßstempel befindlicher, ein definiertes Volumen aufweisender Stababschnitt entweder vollständig aus einer in der Flüssigkeit löslichen Reaktionssubstanz bestehen oder mit dieser beschichtet sein. Dadurch kann eine exakt dosierte Menge der Reaktionssubstanz, und zwar erst während des Mischprozesses, in die Flüssigkeit eingebracht werden. Ein beschichteter Stababschnitt fungiert zudem als Mischhilfe.

Laut Erfindung weist der Ausstoßstempel einen zu einem proximal ausgebildeten Kopfstück hin konisch erweiterten Stempelabschnitt und einen sich zur Ausstoßseite hin erstreckenden geraden Stempelabschnitt mit gleichbleibendem Durchmesser auf. Das Kopfstück ist in einem ersten Führungskanal des Probenahmestabes geführt. Die beiden Stempelabschnitte sind in einem sich zum Probenahmeraum hin konisch verjüngenden zweiten Führungskanal abdichtend geführt, so dass über den hohlen Probenahmestab keine Flüssigkeit nach außen dringen kann. In weiterer Ausgestaltung der Erfindung ist der Mischraum auf der einen Seite durch eine elastische Dicht- und Abstreifmembran mit einer in dieser vorgesehenen Öffnung, in die der konisch verjüngte Probenahmestab abdichtend eingreift, und auf der gegenüberliegenden Seite durch eine sich zu ihrer Austrittsöffnung hin konisch verjüngende Tropfspitze zur Erzeugung von Probentröpfchen mit konstantem Volumen zur Durchführung von Tröpfchentests begrenzt.

Die Austrittsöffnung der Tropfspitze ist durch einen am Boden einer mit dem Aufnahmeröhrchen verschraubbaren Verschlusskappe angeformten Verschlusspfropfen dicht verschließbar. Die Probenahmevorrichtung wird damit höchsten Anforderungen an die Dichtheit, zum Beispiel auch beim Flugzeugtransport, gerecht. Es besteht keine Kontaminationsgefahr bei gefährlichen Erregern. Aber auch nach der Entnahme des Probenahmestabes oder der Entfernung der mit dem Verschlusspfropfen ausgebildeten Verschlusskappe kann die Flüssigkeit über die kleine Öffnung in der Dicht- und Abstreifmembran oder in der Tropfspritze nicht selbsttätig austreten. Erst durch manuellen Druck auf die elastische Wand des Aufnahmeröhrchens gelangt zur Untersuchung in einem Schnelltest ein stets gleich großer Tropfen der Lösung aus dem Mischraum über die konische Tropfspitze nach außen. Für weitere Untersuchungen kann eine bestimmte Probenmenge aus dem Mischraum heraus pipettiert werden oder die Vorrichtung kann an einen Untersuchungsautomaten angeschlossen werden.

In weiterer Ausgestaltung der Erfindung ist in der Tropfspitze oberhalb der Austrittsöffnung ein Rückhalteelement für in der Flüssigkeit verbliebene feste (nicht lösbare) Probenbestandteile oder für die als Mischhilfe fungierenden Teilchen angeordnet. Das Rückhalteelement, das aus einem Sieb oder einem Rost aus mindestens einem Quersteg oder einem offenporigen Schwamm bestehen kann, verhindert, dass die Austrittsöffnung der Tropfspitze durch feste Teilchen ganz oder teilweise zugesetzt wird. Somit ist in jedem Fall die Erzeugung von Probentröpfchen mit konstantem Volumen für den Tröpfchentest gewährleistet. In weiterer Ausbildung der Erfindung ist der am Ende des vorzugsweise aus Kunststoff bestehenden Probenahmestabes vorgesehene Probenahmeraum durch in Umfangsrichtung im Abstand angeordnete Schaufeln begrenzt. Der Probenahmestab einschließlich Probenahmeraum kann aber auch als enge metallische Kanüle ausgeführt sein, die insbesondere zur Aufnahme fester, beispielsweise aus Hartkäse bestehender Lebensmittelproben geeignet ist.

Es versteht sich, dass die Probenahmestäbe entsprechend dem jeweiligen Testverfahren und Probenmaterial einen unterschiedlichen großen kapillaren Probenahmeraum zur Aufnahme einer vorgegebenen Probematerialmenge, beispielsweise 4mg oder 10mg oder 40mg, aufweisen können.

Ein Ausführungsbeispiel der Erfindung wird anhand der Zeichnung, in der
- Fig. 1: eine Seitenansicht einer Vorrichtung zur Entnahme und Aufbereitung einer Probe im Schnitt;
- Fig. 2: eine Seitenansicht des Probenahmestabes;
- Fig. 3: eine Schnittansicht eines Probenahmestabes, je doch ohne Ausstoßstempel; und
- Fig. 4: eine Seitenansicht des Ausstoßstempels
zeigt, näher erläutert.

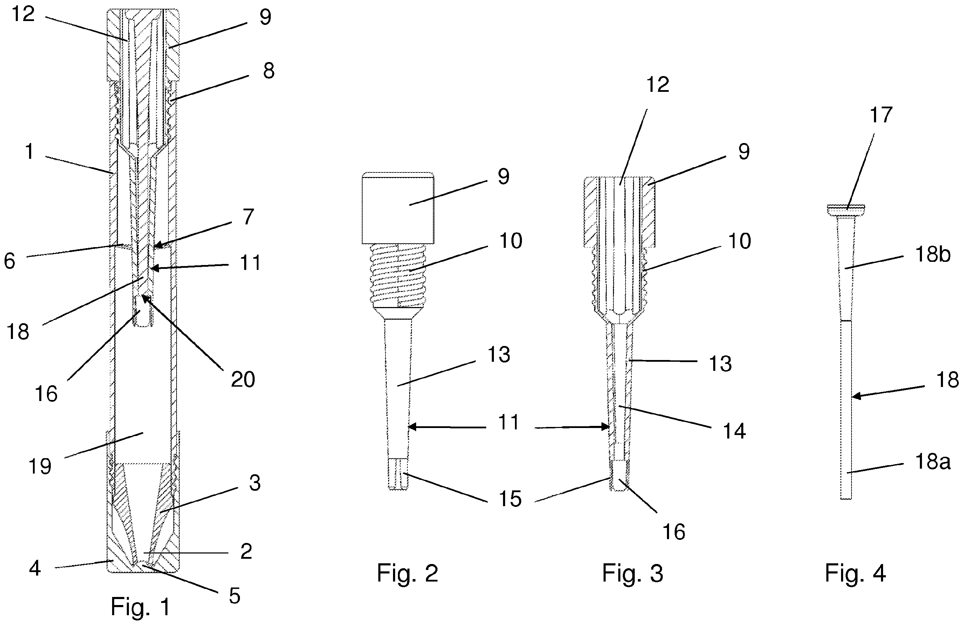

Die in Fig. 1 dargestellte, zur Entnahme und Aufbereitung von Stuhlproben vorgesehene, aber auch zur Probenahme für die Untersuchung von Lebensmitteln wie Fleisch- und Wurstwaren, Milchprodukten und dergleichen geeignete Vorrichtung umfasst ein aus einem verformbaren Kunststoff bestehendes Aufnahmeröhrchen 1 mit einer in dessen distales Ende abdichtend eingesetzten, eine definierte Austrittsöffnung 2 aufweisenden Tropfspitze 3. ein in der Tropfspitze 3 angeordnetes sieb- oder rostartiges Rückhalteelement (nicht dargestellt) verhindert, dass feste Teilchen in den Bereich der Austrittsöffnung 2 gelangen können und die Tröpfchenbildung beeinträchtigen können. Die Austrittsöffnung 2 ist durch eine am Außenumfang des Aufnahmeröhrchens 1 verschraubbare Verschlusskappe 4 verschlossen, indem ein am Boden der Verschlusskappe 4 mittig angeformter, etwa halbkugelförmiger Verschlusspfropfen 5 teilweise in die Austrittsöffnung 2 eingreift. Im Innern des Aufnahmeröhrchens 1 ist eine Dicht- und Abstreifmembran 6 mit einer mittigen Öffnung 7 angeordnet, die zum einen eine Abstreiffunktion und zum anderen eine Abdichtfunktion hat. Am proximalen Ende des Aufnahmeröhrchens 1 ist ein Innengewinde 8 eingeformt, um einen in das Aufnahmeröhrchens 1 ragenden, mit einem Griffstück 9 und an dieses anschließenden Außengewinde 10 versehenen Probenahmestab 11 befestigen zu können. Der Probenahmestab 11 ragt in seinem unteren Bereich durch die Öffnung 7 der Dicht- und Abstreifmembran 6 hindurch in den unterhalb der Membran 6 mit einer Flüssigkeit (Pufferlösung) gefüllten Mischraum 19 des Aufnahmeröhrchens 1. Dieser Mischraum 19 ist auf der einen Seite durch die Dicht- und Abstreifmembran 6 und auf der gegenüberliegenden Seite durch die Verschlusskappe 4 mit dem Verschlusspfropfen 5 dicht verschlossen.

Der als Hohlkörper ausgebildete Probenahmestab 11 weist im Bereich des Griffstücks 9 und des Außengewindes 10 einen ersten weiten Führungskanal 12 auf. An das Außengewinde 10 schließt sich ein sich konisch verjüngender, rohrartiger Stababschnitt 13 mit deutlich kleinerem Außendurchmesser und mit einem zweiten engen, sich konisch verjüngenden Führungskanal 14 an. Der Stababschnitt 13 endet in von dessen freier Stirnseite abstrebenden Schaufein 15, die einen als Kapillare fungierenden, langestreckten Probenahmeraum 16 einschließen. Die Größe des Probenahmeraums 16 kann in Abhängigkeit vom Probematerial und der Art des Testverfahrens bzw. der eingesetzten Testautomaten unterschiedlich sein, um beispielsweise 4mg oder 10mg oder 40mg Probematerial aufzunehmen.

Im Innern des Probenahmestabes 11 ist ein mit einem Kopfstück 17 versehener Ausstoßstempel 18 angeordnet, der das offene distale Ende des Probenahmestabes 11 verschließt und dessen freie Stirnseite den Boden 20 des Probenahmeraums 16 bildet. Über die Länge des ersten - weiten - Führungskanals 12 kann das Kopfstück 17 des Ausstoßstempels 18 verstellt und somit das distale Ende, das heißt der Boden 20 des Probenahmeraums 16 bis weit in den Mischraum 19 verschoben werden.

Nachfolgend wird die Funktion der zuvor beschriebenen Vorrichtung zur Entnahme und Aufbereitung einer Probe beschrieben:
Der Mischraum 19 der in Fig. 1 dargestellten gebrauchsfertigen Vorrichtung ist mit einer Antioxidantien enthaltenden Pufferlösung (nicht dargestellt) gefüllt. Zur Probenahme wird der Probenahmestab 11 aus dem Aufnahmeröhrchen 1 herausgeschraubt und die Spitze des Probenahmestabes 11 vollständig in das Probenmaterial gesteckt und dabei gedreht und anschließend wieder aus dem Probenmaterial herausgezogen. Der Probenahmeraum 16 ist nun - zum Beispiel bei der Stuhlprobenahme - vollständig mit einem sehr harten Stuhl oder auch mit einem weichen bis flüssigen Stuhl gefüllt, und zwar entsprechend der Größe des von den Schaufeln 15 eingeschlossenen Probenahmeraums 16 mit einer bestimmten, für die nachfolgende Untersuchung geeigneten Probematerialmenge. Die Fixierung auch eines sehr weichen bis flüssigen Stuhls im Probenahmeraum ist durch dessen Gestaltung als sehr enger Kapillarraum gewährleistet, in dem ein flüssiger Stuhl durch die Kapillarwirkung gehalten wird und den Probenahmeraum 16 vollständig füllt. Während der Probenahme, das heißt, wenn der Probenahmestab 11 nicht in der Öffnung 7 der Dicht- und Abstreifmembran 6 steckt, kann auch bei einem versehentlichen Umkippen oder Umdrehen des Aufnahmeröhrchens 1 keine Flüssigkeit aus dem Mischraum 19 auslaufen, da aufgrund der Oberflächenspannung über die sehr kleine Öffnung 7 in der Dicht- und Abstreifmembran 6 keine Flüssigkeit auslaufen kann.

Nach der Probenahme wird der Probennahmestab 11 wieder in das Aufnahmeröhrchen 1 eingeführt. Dabei wird der vordere (distale) Abschnitt des konisch verjüngten Probenahmestabes 11 durch die Öffnung 7 in der elastischen Dicht- und Abstreifmembran 6 geführt. Der Rand der Öffnung 7, deren Durchmesser kleiner als der Außendurchmesser des Probenahmestabes 11 an dessen distalem Ende ist, wird elastisch an den Außenumfang des Probenahmestabes 11 gepresst, so dass an dessen Außenfläche haftende Probenreste abgestreift werden und in Verbindung mit der abdichtenden Verschraubung (8, 10) des Probenahmestabes 11 im Aufnahmeröhrchen 1 außerdem eine sichere Abdichtung am proximalen Ende der Vorrichtung gewährleistet ist. Die Abdichtung zwischen dem Mischraum 19 und dem konisch verjüngten zweiten Führungskanal 14 ist durch dessen elastische Wirkung auf den Ausstoßstempel 18 sichergestellt, wobei die Abdichtung noch dadurch verbessert wird, dass sich an einen unteren Abschnitt 18a des Ausstoßstempels mit konstantem Durchmesser ein sich zum Kopfstück 17 hin konisch erweiterter Abschnitt 18b anschließt. Die sichere Abdichtung am distalen Ende erfolgt über die Verschlusskappe 4 und den in dieser ausgebildeten, an der Austrittsöffnung 2 der Tropfspitze 3 elastisch wirkenden Verschlusspfropfen 5.

Um eine vollständige Auflösung der Probe zu bewirken, wird der Ausstoßstempel 18 nach unten gedrückt, so dass die Probe in die im Mischraum 19 befindliche Flüssigkeit gelangt und sich leicht lösen kann. Eine noch bessere Auflösung der Probe kann dadurch bewirkt werden, dass das freie, an den Probenahmeraum 16 angrenzende Ende des unteren Abschnitts 18a des Ausstoßstempels 18 aus einem als Dichtelement wirkenden Kügelchen (nicht dargestellt) besteht, dessen Durchmesser dem des Abschnitts 18a entspricht. Bei einer Schüttelbewegung des Aufnahmeröhrchens 1 kann die - gegebenenfalls noch an dem Kügelchen haftende - Probe noch besser abgelöst und aufgelöst werden. Das Kügelchen kann zudem mit einer Reaktionssubstanz beschichtet sein, die sich während des Mischvorgangs in der Flüssigkeit löst und die Probe weiter für die anschließende Untersuchung aufbereitet. Zwischen dem freien Ende des Ausstoßstempels und dem - in diesem Fall unbeschichteten Kügelchen kann auch ein Stababschnitt angeordnet sein, der entweder vollständig aus einer sich nach dem Ausstoßen in der Flüssigkeit lösenden, genau bemessenen Reaktionssubstanz besteht oder mit einer derartigen Substanz beschichtet ist.

Mit der zuvor beschriebenen Vorrichtung und der in dieser aufbereiteten Probe können - aufgrund der gleichbleibenden Größe der Tropfspitze 3 bei konstanter Tropfengröße - wiederholt qualitative Tropfentests, aber auch Pipettiertests und Automatentests durchgeführt werden. Die Vorrichtung ist für die Entnahme und Aufbereitung flüssiger bis sehr fester Stuhlproben und gleichermaßen für Lebensmittel geeignet.

In der oben beschriebenen Ausführungsform besteht die Probenahmevorrichtung aus Kunststoff. Für die Entnahme sehr fester Lebensmittelproben kann der Probenahmestab mit dem Probenahmeraum, der in diesem Fall nicht von Schaufeln gebildet ist, als metallische Kanüle ausgeführt sein.

### Bezugszeichenliste

- 1: Aufnahmeröhrchen
- 2: Austrittsöffnung von 3
- 3: Tropfspitze von 1
- 4: Verschlusskappe
- 5: Verschlusspfropfen von 4
- 6: Dicht- und Abstreifmembran
- 7: Öffnung von 6
- 8: Innengewinde von 1
- 9: Griffstück von 11
- 10: Außengewinde von 11
- 11: Probenahmestab
- 12: erster Führungskanal von 11
- 13: konischer Stababschnitt von 11
- 14: zweiter Fülhrungskanal von 11
- 15: Schaufeln von 16
- 16: Probenahmeraum, Kapillarraum von 11
- 17: Kopfstück von 18
- 18: Ausstoßstempel
- 18a: gerader unterer Abschnitt von 18
- 18b: konisch erweiterter oberer Abschnitt von 18
- 19: Mischraum von 1
- 20: Stirnseite von 18, Boden von 16

## Patentansprüche

1. Vorrichtung zur Entnahme und Aufbereitung einer Probe zur qualitativen und quantitativen Bestimmung von in dieser enthaltenen Substanzen, insbesondere einer Stuhlprobe für die Stuhluntersuchung, die einen Probenahmestab (11) mit einer als Probenahmeraum (16) ausgebildeten Entnahmestruktur zur Aufnahme einer bestimmten Probemenge und ein den Probenahmestab (11) aufnehmen des Aufnahmeröhrchen (1), das einen mit einer Flüssigkeit gefüllten Mischraum (19) zur Konservierung, Auflösung und Aufbereitung der in dem Probenahmeraum (16) befindlichen Probe aufweist, umfasst, wobei der Probenahmestab (11) als beidseitig offener Hohlkörper mit einem in diesem verschiebbar und abdichtend geführten Ausstoßstempel (18) zum Ausstoßen der Probe aus dem Probenahmeraum (16) in den Mischraum (19) ausgebildet ist, wobei der Probenahmeraum (16) als Kapillare ausgebildet ist und die Aufnahme und das Halten von flüssigem Probenmaterial ermöglicht und aus vom stirnseitigen Rand des Probenahmestabes (11) abstrebenden Schaufeln (15) besteht
wobei
der Ausstoßstempel (18) einen zu einem proximal ausgebildeten Kopfstück (17) hin konisch erweiterten Stempelabschnitt (18b) und einen geraden Stempelabschnitt (18a) mit gleichbleibendem Durchmesser aufweist, und das stirnseitige distale Ende des Ausstoßstempels (18) den Boden des Probenahmeraums (16) bildet
**dadurch gekennzeichnet, dass**
das Kopfstück (17) in einem ersten Führungskanal (12) des Probenahmestabes (11) und die Stempelabschnitte (18a, 18b) in einem sich zum Probenahmeraum (16) hin konisch verjüngenden zweiten Führungskanal (14) abdichtend geführt sind; und
wobei über die Länge des ersten Führungskanals (12) das Kopfstück (17) des Ausstoßstempels (18) verstellbar ist, so dass das stirnseitige distale Ende des Ausstoßstempels (18) in den Mischraum verschoben wird.

2. Vorrichtung nach Anspruch 1, **dadurch gekennzeichnet, dass** am distalen Ende des Ausstoßstempels (18) mindestens ein - zunächst als Dichtelement und nach dem Ausstoßen der Probe in den Mischraum (19) als Mischhilfe fungierendes - Kügelchen angeordnet ist.

3. Vorrichtung nach Anspruch 2, **dadurch gekennzeichnet, dass** das Kügelchen mit einer in der Flüssigkeit löslichen Reaktionssubstanz beschichtet ist

4. Vorrichtung nach Anspruch 2, **dadurch gekennzeichnet, dass** zwischen dem Ausstoßstempel (18) und dem als Dichtelement fungierenden Kügelchen ein mit einer in der Flüssigkeit löslichen Reaktionssubstanz beschichteter oder vollständig aus der Reaktionssubstanz bestehender Stababschnitt angeordnet ist.

5. Vorrichtung nach Anspruch 1, **dadurch gekennzeichnet, dass** der Probenahmestab (11) mit Probenahmeraum (16) als enge metallische Kanüle ausgebildet ist.

6. Vorrichtung nach Anspruch 1, **dadurch gekennzeichnet, dass** der Mischraum (19) auf der einen Seite durch eine elastische Dicht- und Abstreifmembran (6) mit einer Öffnung (7), in die der konisch verjüngte Probenahmestab (11) abdichtend eingreift, und auf der gegenüberliegenden Seite durch eine sich zu ihrer Austrittsöffnung (2) hin konisch verjüngende Tropfspitze (3) zur Erzeugung von Probentröpfchen mit konstantem Volumen begrenzt ist.

7. Vorrichtung nach Anspruch 6, **dadurch gekennzeichnet, dass** in der Tropfspitze (3) oberhalb derAustrittsöffnung (2) ein Rückhalteelementfür im Mischraum befindliche feste Teilchen angeordnet ist.

8. Vorrichtung nach Anspruch 6, **dadurch gekennzeichnet, dass** die Austrittsöffnung (2) durch einen am Boden einer mit dem Aufnahmeröhrchen (1) verschraubbaren Verschlusskappe (4) angeformten Verschlusspfropfen (5) dicht verschließbar ist.

## Claims

1. A device for taking and processing a sample, in particular a stool sample for stool examination, in order to qualitatively and quantitatively determine substances contained therein, said device comprising a sampling rod (11) with a sampling structure, designed as a sampling chamber (16), for accommodating a specific amount of sample, and an accommodating tube (1) accommodating the sampling rod (11), said accommodating tube having a mixing chamber (19), filled with a liquid, for preserving, dissolving, and processing the sample located in the sampling chamber (16), the sampling rod (11) being designed as a hollow body that is open at both ends with an ejecting plunger (18) guided therein in a movable and sealing manner for ejecting the sample from the sampling chamber (16) into the mixing chamber (19), wherein the sampling chamber (16) is designed as a capillary and allows accommodation and holding of liquid sample material and consists of blades (15) projecting from the frontal edge of the sampling rod (11),
wherein the ejecting plunger (18) has a plunger portion (18b) conically expanded towards a proximally formed head part (17), and a straight plunger portion (18a) with a constant diameter, and wherein the frontal distal end of the ejecting plunger (18) forms the bottom of the sampling chamber (16),
**characterized in that** the head part (17) is sealingly guided in a first guiding channel (12) of the sampling rod (11) and the plunger portions (18a, 18b) are sealingly guided in a second guiding channel (14) that is conically tapered towards the sampling chamber (16); and
wherein the head part (17) of the ejecting plunger (18) is repositionable over the length of the first guiding channel (12) such that the frontal distal end of the ejecting plunger (18) is displaced into the mixing chamber.

2. The device of claim 1, **characterized in that** there is arranged at the distal end of the ejecting plunger (18) at least one bead which functions initially as a sealing element, and as a mixing aid after the sample has been ejected into the mixing chamber (19).

3. The device of claim 2, **characterized in that** the bead is coated with a reaction substance which is soluble in the liquid.

4. The device of claim 2, **characterized in that** there is arranged, between the ejecting plunger (18) and the bead functioning as a sealing element, a rod portion coated with the reaction substance that is soluble in the liquid, or consisting entirely of the reaction substance.

5. The device of claim 1, **characterized in that** the sampling rod (11) with the sampling chamber (16) is designed as a narrow metallic cannula.

6. The device of claim 1, **characterized in that** the mixing chamber (19) is bounded on one side by an elastic sealing and scraping membrane (6) that has an opening (7) with which the conically tapered sampling rod (11) sealingly engages, and on the opposite side by a drip tip (3), which is conically tapered towards its outlet opening (2), for generating sample droplets with a constant volume.

7. The device of claim 6, **characterized in that** there is arranged, in the drip tip (3) above the outlet opening (2), a retaining element for solid particles present in the mixing chamber.

8. The device of claim 6, **characterized in that** the outlet opening (2) is tightly sealable by a sealing plug (5) integrally formed on the bottom of a sealing cap (4) that can be screwed on the accommodating tube (1).

## Revendications

1. Dispositif destiné au prélèvement et à la préparation d'un échantillon en vue de la détermination qualitative et quantitative de substances contenues dans celui-ci, notamment d'un échantillon de selles pour l'analyse des selles, qui comprend une tige de prélèvement d'échantillon (11) avec une structure de prélèvement constituée comme espace de prélèvement d'échantillon (16) en vue de la réception d'un certain volume de prélèvement et une éprouvette de réception (1) recevant la tige de prélèvement d'échantillon (11), qui présente un espace de mélange (19) rempli d'un liquide en vue de la conservation, de la dissolution et de la préparation de l'échantillon se trouvant dans l'espace de prélèvement d'échantillon (16), sachant que la tige de prélèvement d'échantillon (11) est constituée comme corps creux ouvert des deux côtés avec une estampille d'éjection (18) guidée dans celle-ci de manière décalable et étanche en vue de l'éjection de l'échantillon hors de l'espace de prélèvement d'échantillon (16) dans l'espace de mélange (19), sachant que l'espace de prélèvement d'échantillon (16) est constitué comme vaisseau capillaire, permet la réception et la retenue de matière d'échantillon liquide et se compose de pelles (15) s'étendant à partir du bord frontal de la tige de prélèvement d'échantillon (11),
sachant que l'estampille d'éjection (18) présente une section d'estampille élargie coniquement (18b) vers un élément de tête formé proximalement (17) et une section d'estampille droite (18a) à diamètre constant, et que l'extrémité distale frontale de l'estampille d'éjection (18) constitue le fond de l'espace de prélèvement d'échantillon (16),
**caractérisé en ce que**
l'élément de tête (17) est guidé dans un premier canal de guidage (12) de la tige de prélèvement d'échantillon (11) et les sections d'estampille (18a, 18b) le sont de manière étanche dans un second canal de guidage (14) s'amincissant coniquement vers l'espace de prélèvement d'échantillon (16) ; et
sachant que via la longueur du premier canal de guidage (12), l'élément de tête (17) de l'estampille d'éjection (18) est réglable, de sorte que l'extrémité distale frontale de l'estampille d'éjection (18) est décalée dans l'espace de mélange.

2. Dispositif selon la revendication 1, **caractérisé en ce que**, sur l'extrémité distale de l'estampille d'éjection (18), au moins une bille - fonctionnant d'abord comme élément d'étanchéité et après l'éjection de l'échantillon dans l'espace de mélange (19) comme agent de mélange - est disposée.

3. Dispositif selon la revendication 2, **caractérisé en ce que** la bille est enrobée d'une substance réactive soluble dans le liquide.

4. Dispositif selon la revendication 2, **caractérisé en ce que**, entre l'estampille d'éjection (18) et la bille fonctionnant comme élément d'étanchéité, une section de tige enrobée avec une substance réactive soluble dans le liquide ou composée entièrement de la substance réactive est disposée.

5. Dispositif selon la revendication 1, **caractérisé en ce que** la tige de prélèvement d'échantillon (11) est constituée avec son espace de prélèvement d'échantillon (16) comme une canule métallique étroite.

6. Dispositif selon la revendication 1, **caractérisé en ce que** l'espace de mélange (19) est limité d'un côté par une membrane élastique d'étanchéité et de raclage (6) avec un orifice (7) dans lequel la tige de prélèvement d'échantillon (11) s'amincissant coniquement prend, et du côté situé en face par une pointe d'égouttement (3) s'amincissant coniquement vers son orifice de sortie (2) en vue de la production de gouttelettes d'échantillon à volume constant.

7. Dispositif selon la revendication 6, **caractérisé en ce que**, dans la pointe d'égouttement (3) un élément de retenue est disposé au-dessus de l'orifice de sortie (2) pour les particules solides se trouvant dans l'espace de mélange.

8. Dispositif selon la revendication 6, **caractérisé en ce que** l'orifice de sortie (2) est fermable de manière étanche par un bouchon de fermeture (5) formé sur le fond d'un chapeau de fermeture (4) vissable avec l'éprouvette de réception (1).
